# FASCICULE DE BREVET EUROPEEN

(11) **EP 4 085 160 B1**
(45) Date de publication et mention de la délivrance du brevet: **25.02.2026**
(21) Numéro de dépôt: 20851217.8
(22) Date de dépôt: 19.12.2020
(51) Int. Cl.: C23F 13/04, G01N 17/02, G01N 27/30, G01N 33/24, G01N 27/333, G01N 27/416

(54) **DISPOSITIF A CAPTEUR DE PH DESTINE A ETRE INSERE DANS LE SOL, PROCEDE DE MESURE DU PH, EN PARTICULIER POUR LA PROTECTION CATHODIQUE**
PH-SENSORVORRICHTUNG ZUM EINBRINGEN IN DEN BODEN, VERFAHREN ZUM MESSEN DES PH-WERTES, INSBESONDERE FÜR DEN KATHODISCHEN SCHUTZ
PH SENSOR DEVICE FOR INSERTION INTO THE GROUND, METHOD FOR MEASURING THE PH, IN PARTICULAR FOR CATHODIC PROTECTION

(30) Priorité: 30.12.2019 FR 1915721
(43) Date de publication de la demande: 09.11.2022
(73) Titulaire: GRTgaz, 92270 Bois-Colombes (FR); Centre national de la recherche scientifique, 75016 Paris (FR); SORBONNE UNIVERSITE, 75006 Paris (FR)
(72) Inventeur: DEBIEMME-CHOUVY, Catherine, 94100 SAINT-MAUR-DES-FOSSES (FR); FAKHRY, Ahmed, 94600 CHOISY-LE-ROI (FR); FLEURY, Elisabeth, 95120 ERMONT (FR)
(74) Mandataire: Cabinet Beau de Loménie
(86) Numéro de dépôt international: PCT/FR2020/052575
(87) Numéro de publication internationale: WO 2021/136905

(56) Documents cités:
- EP-B1- 2 602 609
- WO-A1-2017/030934
- CN-A- 1 936 562
- DE-A1- 102012 017 415
- KR-A- 20190 083 120
- US-A1- 2007 298 278
- US-A1- 2019 368 054
- YUQING M ET AL: "New technology for the detection of pH", JOURNAL OF BIOCHEMICAL AND BIOPHYSICAL METHODS, AMSTERDAM, NL, vol. 63, no. 1, 29 April 2005 (2005-04-29), pages 1 - 9, XP027670191, ISSN: 0165-022X, [retrieved on 20050429]
- PETER KURZWEIL: "Metal Oxides and Ion-Exchanging Surfaces as pH Sensors in Liquids: State-of-the-Art and Outlook", SENSORS, vol. 9, no. 6, 23 June 2009 (2009-06-23), pages 4955 - 4985, XP055176863, DOI: 10.3390/s90604955

## Description

### Domaine Technique

La présente invention concerne le domaine de la détermination de caractéristiques du sol. En particulier, elle concerne la détermination du pH d'une région du sol dans laquelle une canalisation peut être installée et pour laquelle on met en œuvre une protection cathodique.

### Technique antérieure

Les canalisations enterrées dans le sol et qui transportent des fluides (typiquement du gaz) sont généralement en acier avec une composition qui rend la corrosion possible. Cette corrosion est généralement associée au pH du sol, ou plus précisément au pH d'un fluide contenu dans le sol (par exemple de l'eau).

On connait pour différents matériaux des diagrammes appelés diagrammes de Pourbaix. Ces diagrammes permettent de placer un matériau dans différentes zones qui illustrent le risque de corrosion (typiquement corrosion, passivation, immunité) en fonction de deux paramètres : le pH du fluide en contact avec le matériau et le potentiel électrique appliqué au matériau. Il en découle que l'on applique généralement un potentiel électrique aux canalisations pour qu'elles puissent se trouver dans la zone dans laquelle il y a une réduction de la vitesse de corrosion suffisamment faible pour respecter les normes en vigueur. Les méthodes dans lesquelles on applique un potentiel électrique à une canalisation s'appellent des méthodes de protection cathodique.

La norme NF EN ISO 15589-1 impose de maintenir le potentiel électrique des ouvrages métalliques enterrés en dessous de -0,85V/Cu-CuSO₄. En pratique, le potentiel électrique des canalisations est maintenu à environ -1V/Cu-CuSO₄.

Comme on le conçoit, le pH des fluides contenus dans le sol peut varier en fonction de nombreux paramètres, et ce pH peut même être influencé par la protection cathodique elle-même.

Dès lors, la connaissance de la valeur du pH du fluide qui entoure une canalisation est critique.

Il n'est pas envisageable de mettre en œuvre une excavation pour atteindre une canalisation enterrée et mesurer une valeur de pH, compte tenu du coût que cela représente.

Il a été proposé de placer des papiers pH dans le sol, à proximité des canalisations mais pas à la même profondeur. Cette méthode ne permet pas d'avoir une valeur précise du pH du sol mais uniquement un ordre de grandeur. En outre, elle est subjective car elle dépend de l'appréciation de la couleur du papier par l'opérateur, couleur qui peut-être faussée par la couleur même du sol. Enfin, une mesure dans laquelle on utilise du papier pH est ponctuelle et ne permet pas d'avoir une connaissance du pH en continu.

La méthode dite du Lixiviat propose de prélever une portion du sol, de la diluer, et de mesurer le pH de la solution obtenue. Cette méthode fournit une approximation du pH mais celle-ci n'est pas suffisamment précise puisque le milieu est dilué, ce qui modifie forcément la valeur réelle du pH du milieu. Cette méthode a également pour inconvénient de n'être réalisable qu'en laboratoire et à l'aide d'une sonde pH classique (qui ne fonctionne généralement uniquement qu'en milieu liquide).

On connait également un capteur pH commercialisé par la société américaine M.C. Miller CO. Inc. qui utilise une électrode à base d'antimoine. Ce capteur pH présente des inconvénients. Tout d'abord, l'antimoine est toxique et cancérigène, ce qui pose des problèmes pour la sécurité des utilisateurs. Ensuite, ce capteur fournit une valeur de potentiel de circuit ouvert qui est corrélée à une valeur de pH, mais cette corrélation n'est possible qu'à partir d'une calibration réalisée en amont dans un milieu inconnu. De ce fait, il subsiste une incertitude sur cette mesure car le pH d'un milieu à l'autre varie en fonction des espèces présentes dans le milieu.

Le document WO2008031453 décrit un procédé pour déterminer les propriétés de passivation d'un mélange contenant au moins deux composants (du ciment et de l'eau). Dans ce document, la mesure de la passivation sur la surface d'un acier de référence permet de détecter la présence d'eau, de substances agressives, une teneur en chlorure trop élevée ou encore un pH trop bas.

De l'état de la technique antérieure, on connait les documents EP 2 602 609, US 2007/298278, US 2019/368054, CN 1 936 562, et « Metal Oxides and lon-Echanging Surfaces as pH sensors in Liquids : State-of-the-Art and Outlook » (Peter Kurzweil, SENSORS, Vol. 9, No. 6, 23 juin 2009, pages 4955-4985).

L'invention vise à résoudre certains au moins des inconvénients précités.

### Exposé de l'invention

À cet effet, l'invention propose un procédé selon la revendication 1.

Ce procédé sera préférentiellement mis en œuvre à proximité d'une canalisation réellement enterrée, par exemple au-dessus de cette canalisation.

Par exemple, on pourra mettre en œuvre ce procédé près d'une station de mesure (ou de prise) du potentiel électrique de la canalisation généralement agencée au-dessus des canalisations. Ces stations de mesure sont bien entendu placées à la surface du sol. Dans ce cas, l'invention permet avantageusement d'appliquer le même potentiel électrique à l'élément métallique que celui qui est appliqué à la canalisation (on le prélève directement sur la canalisation). On obtient ainsi une mesure encore plus réaliste du pH au niveau de la canalisation enterrée.

Les inventeurs de la présente invention ont observé qu'il est possible d'utiliser des matériaux polymères pour réaliser des capteurs de pH, et qu'en associant ces capteurs à un élément métallique recevant un potentiel électrique, on peut mettre en œuvre une mesure à faible profondeur dans un environnement proche de celui d'une canalisation enterrée qui reçoit elle aussi un potentiel électrique de protection cathodique. La mesure du pH par le capteur illustre donc bien une mesure de pH qui pourrait être faite au voisinage proche d'une canalisation (par exemple à quelques dizaines de centimètres d'une canalisation)

L'utilisation de polymères permet par ailleurs d'obtenir un capteur robuste, qui peut être utilisé dans le sol en ayant une bonne durée de vie. Aussi, ces polymères n'ont pas les inconvénients des capteurs qui contiennent de l'antimoine qui sont dangereux pour les utilisateurs. On choisira préférentiellement des polymères contenant des groupes susceptibles d'attirer les protons, de sorte que le potentiel électrique à la surface sera affecté.

Typiquement, le potentiel électrique est un potentiel électrique ayant une valeur qui pourrait être appliquée à une canalisation (par exemple inférieur à -0,85V/Cu-CuSO₄).

Pour appliquer ce potentiel électrique, on pourra utiliser un des moyens de connexion électrique comportant un câble.

Aussi, la surface comprenant un matériau polymère capable d'attirer les protons peut être agencée de manière à recouvrir au moins partiellement une autre surface du capteur de pH, par exemple une surface d'un autre élément en métal qui sera conducteur et pourra transmettre le signal résultant de la présence de protons sur la surface comprenant un matériau polymère.

Selon un mode de réalisation particulier, le polymère contient un ou plusieurs groupes aminos (dans leur chaîne moléculaire).

Les inventeurs ont observé que les groupes aminos fonctionnent bien pour la formation de capteurs de pH.

A titre indicatif, le polymère peut être un polymère conducteur.

Selon un mode de réalisation particulier, le polymère contenant des groupes aminos est choisi dans le groupe comprenant le polypyrrolle et le poly(3,4-éthylènedioxythiophène).

Ces deux matériaux polymères sont conducteurs.

Selon l'invention, le dispositif comprend un corps muni d'au moins une première ouverture configurée pour laisser, lorsque le dispositif est inséré dans le sol, le fluide contenu dans ladite région être en contact avec ladite surface comprenant un matériau polymère et avec ladite surface de l'élément métallique, et au moins une deuxième ouverture configurée pour recevoir lesdits moyens de connexion électrique.

Ce corps peut comporter un matériau à base d'un polymère que l'on choisira pour ses propriétés mécaniques et sa résistance aux fluides avec lesquels le corps pourra être en contact dans le sol.

À titre indicatif, la première ouverture peut être l'ouverture basse du corps, orientée en bas quand le dispositif est inséré dans le sol. La deuxième ouverture sera orientée vers le haut pour laisser un passage aux moyens de connexion électrique.

Selon l'invention, ladite surface comprenant un matériau polymère et ladite surface de l'élément métallique sont agencées en retrait par rapport à une surface externe du corps où sont formées les ouvertures.

Ce retrait permet de protéger ces deux surfaces.

Par exemple, ces deux surfaces peuvent être en retrait d'un millimètre.

Selon un mode de réalisation particulier, le dispositif comprend des premiers moyens de maintien de l'étanchéité agencés à l'interface entre d'une part ladite surface comprenant un matériau polymère et ladite surface de l'élément métallique et d'autre part le corps, pour empêcher le fluide contenu dans ladite région de pénétrer à l'intérieur du corps.

Il a été observé qu'il est particulièrement important de maintenir l'étanchéité du dispositif dès lors qu'il est en contact avec les fluides présents dans le sol. Les moyens de création d'étanchéité pourront par exemple comprendre un ou plusieurs joints.

Selon un mode de réalisation particulier, le dispositif comprend des deuxièmes moyens de maintien de l'étanchéité agencés à l'interface entre d'une part les moyens de connexion électrique et d'autre part le corps, pour empêcher le fluide contenu dans ladite région de pénétrer à l'intérieur du corps.

De la même manière, ces deuxièmes moyens de création d'étanchéité peuvent comprendre un ou plusieurs joints et/ou un presse-étoupe.

Selon un mode de réalisation particulier, le capteur de pH et/ou l'élément métallique sont amovibles par rapport au corps.

Il peut être particulièrement intéressant de pouvoir changer ces éléments indépendamment d'autres éléments du dispositif.

Par amovible, on entend que le capteur de pH et/ou l'élément métallique peuvent être retirés soit sans outils (par exemple s'ils sont fixés par clipsage), soit avec un outil (par exemple s'ils sont fixés par vissage).

Les moyens de connexion électrique peuvent être des moyens connectables et dé-connectables, par exemple comprenant des fiches.

Selon un mode de réalisation particulier, ladite surface comprenant un matériau polymère et ladite surface de l'élément métallique sont espacées par une bande ayant une épaisseur comprise entre 0,5 et 1,5 millimètre, par exemple égale à 1 millimètre.

Cette bande permet d'éviter tout contact électrique entre l'élément métallique et la surface en polymère. Préférentiellement, on utilisera une bande ayant une épaisseur d'1 millimètre pour que la présence de l'élément métallique puisse bien illustrer l'environnement dans lequel se trouve une canalisation enterrée.

Selon un mode de réalisation particulier, ladite surface de l'élément métallique a une forme annulaire et ladite surface comprenant un matériau polymère est agencée de manière à être entourée par ladite surface de l'élément métallique.

Il a été observé par les inventeurs de la présente invention que cet agencement, dans lequel la surface comprenant un matériau polymère est entourée par la surface de l'élément métallique illustre bien la proximité d'un capteur avec une canalisation, et permet en particulier d'obtenir une meilleure sensibilité aux changements de pH du milieu en contact avec la surface du matériau polymère.

Selon un mode de réalisation particulier, ladite surface de l'élément métallique a une aire au moins supérieure ou égale à 1 cm² et ladite surface comprenant un matériau polymère a une aire au moins supérieure ou égale à 0,07cm².

Selon un mode de mise en œuvre particulier, le procédé comprend :
- une mise en œuvre du procédé de mesure du pH tel que défini ci-avant pour une région du sol située au-dessus d'une canalisation,
- une détermination d'une nouvelle valeur de potentiel électrique à appliquer à ladite canalisation à partir du résultat de ladite mesure du pH.

Ainsi, l'invention permet d'améliorer la protection cathodique des canalisations enterrées, sans qu'il ne soit nécessaire de mettre en œuvre des mesures directement au niveau des canalisations enterrées.

À titre indicatif, la détermination de la nouvelle valeur de potentiel électrique peut être réalisée en partie automatiquement, par exemple par un programme d'ordinateur exécuté par un processeur du dispositif ou connecté au dispositif, et sur la base de données enregistrées dans une mémoire du dispositif ou d'un autre dispositif telles qu'un diagramme de Pourbaix.

Par exemple, ce programme d'ordinateur peut délivrer une gamme de nouvelles valeurs possible, ou une unique valeur possible.

L'invention propose également un dispositif selon la revendication 11.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :
[Fig. 1] La figure 1 est un exemple de diagramme de Pourbaix.
[Fig. 2] La figure 2 est une vue en coupe d'un premier exemple de dispositif.
[Fig. 3] La figure 3 est une vue de dessous du premier exemple de dispositif.
[Fig. 4] La figure 4 est une vue en éclaté du premier exemple de dispositif.
[Fig. 5] La figure 5 est une vue en coupe d'un deuxième exemple de dispositif.
[Fig. 6] La figure 6 est une vue de dessous du deuxième exemple de dispositif.
[Fig. 7] La figure 7 est une vue en éclaté du deuxième exemple de dispositif.
[Fig. 8] La figure 8 illustre des stations de prise de potentiel électrique utilisée dans un exemple au-dessus d'une canalisation.

### Description des modes de réalisation

On va maintenant décrire des dispositifs qui permettent de mesurer le pH de fluides contenus dans le sol, qui peuvent être insérés dans le sol à faible profondeur, et qui obtiennent une valeur qui illustre bien celle qui pourrait être mesurée à proximité d'une canalisation enterrée plus profonde.

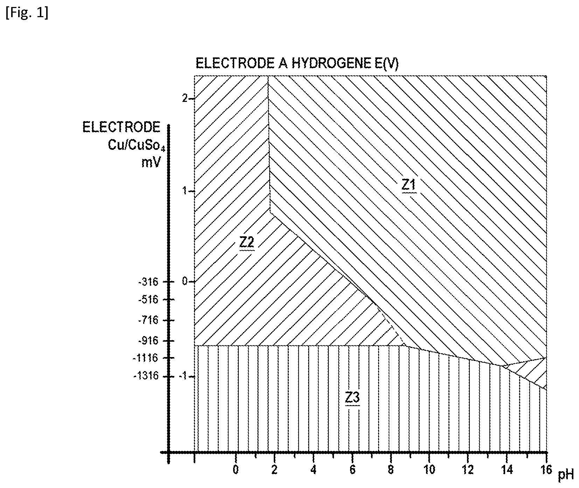

La figure 1 est un exemple de diagramme de Pourbaix, qui montre dans quelle zone se trouve un métal en contact avec un fluide, en fonction du pH de ce fluide et du potentiel électrique appliqué au métal.

On retrouve notamment trois zones principales : la zone de passivation Z1, la zone de corrosion Z2, et la zone d'immunité Z3. Généralement, on applique un potentiel électrique pour qu'un métal se retrouve dans une zone où le risque de corrosion est faible ou encore où la vitesse de corrosion est faible..

En cas de variation (baisse ou hausse) du pH, il peut donc être intéressant de déterminer une nouvelle valeur de potentiel électrique à appliquer, par exemple automatiquement par lecture dans une cartographie basée sur un diagramme de Pourbaix.

Aussi, il a été observé que l'application d'un potentiel électrique sur un métal peut avoir un effet sur la valeur du pH.

L'utilisation d'un simple capteur de pH à faible profondeur n'est donc pas suffisante pour déterminer la valeur du pH du fluide en contact avec une canalisation entourée.

On peut noter que généralement, les canalisations sont enterrées à des profondeurs minimales de 80 cm. Avec l'invention qui sera décrite ci-après, on peut mettre en œuvre une mesure à faible profondeur, typiquement de l'ordre de 20 à 50 cm, mais en obtenant néanmoins une valeur de pH qui illustre bien celle que l'on pourrait mesurer à proximité de la canalisation, par exemple à une distance inférieure à une dizaine de cm.

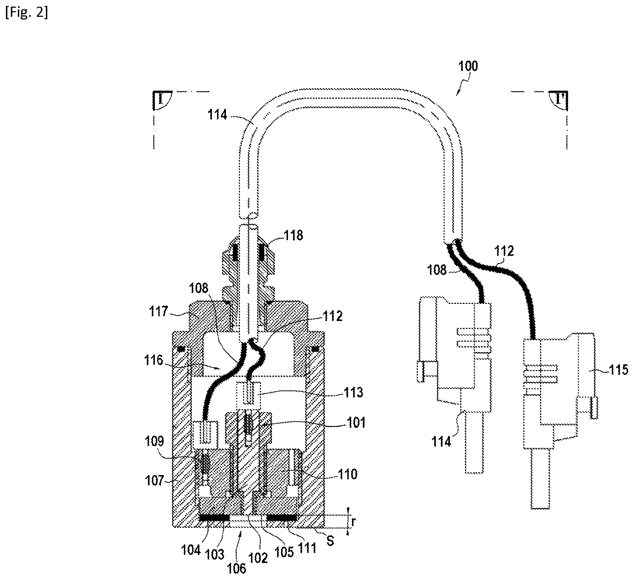

Sur la figure 2, un dispositif 100 à capteur de pH a été représenté en coupe. Ce dispositif est destiné à être inséré dans le sol, par exemple à une profondeur de l'ordre de 20 à 30 cm, et avantageusement au-dessus d'une canalisation pour laquelle on souhaite vérifier l'état de la corrosion. Encore plus précisément, on peut insérer ce dispositif dans le sol à proximité d'une station de mesure du potentiel électrique (ou prise de potentiel) de la canalisation généralement agencée au-dessus des canalisations.

Ce dispositif 100 comporte un capteur de pH 101 comprenant une électrode ayant une surface 102 qui comporte du polypyrrole située en bas sur la figure. L'invention n'est néanmoins pas limitée à l'utilisation du polypyrrole et concerne également l'utilisation d'autres polymères conducteurs, comme par exemple le poly(3,4-éthylènedioxythiophène), également désigné sous le nom de PEDOT.

L'invention n'est néanmoins pas limitée à l'utilisation de ces deux matériaux et peut être mise en œuvre avec tout polymère capable d'attirer les protons, par exemple des polymères comprenant un ou plusieurs groupes aminos.

L'obtention d'une couche de polypyrrole peut être réalisée en suivant la méthode décrite dans la thèse « Synthèse par voie électrochimique de nanostructures de polymères conducteurs sans emploi d'une matrice support » (Ahmed Fakhry, 2014, Université Pierre et Marie Curie).

Plus précisément, on pourra former un film de polypyrrole par voie électrochimique sur un support qui peut être soit de l'acier inoxydable, du platine, de l'or, etc. Ce support porte la référence 103 sur la figure et forme, avec la surface 102, une électrode du capteur de pH 101. Ainsi, le polypyrrole recouvre la surface du support pour former la surface externe 102.

La formation ou synthèse se fait dans une cellule à trois électrodes contenant une solution avec un monomère pyrrole, un dopant tel que du perchlorate (ClO₄⁻) et un électrolyte support, par exemple le K₂HPO₄.

Ceci permet d'obtenir des nanostructures de polypyrrole qui sont orientées vers l'extérieur du capteur, vers le bas sur la figure.

On peut noter que le polypyrrole est un polymère conducteur qui présente dans sa structure moléculaire des groupes dits « amino » qui ont une affinité avec les protons présents dans le milieu en contact avec le polypyrrolle (par exemple un fluide). La réaction des protons avec les groupes « amino » crée ainsi un excès de densité de charges locales à la surface de l'électrode contenant du polypyrrole. La réponse mesurée par une variation de potentiel électrique peut ainsi être considérée comme étant un comportement contrôlé par une réaction de surface qui se produit sur le film de polymère.

On peut également noter que l'utilisation du polypyrrole comme matériau pour équiper une surface d'un capteur de pH. Le polypyrrole est un matériau respectueux de l'environnement, facile à synthétiser et peu onéreux.

Par ailleurs, on notera qu'il est possible de mettre en œuvre une étape de calibration du capteur obtenu. On peut mettre en œuvre cette calibration en milieu liquide ou même dans le sol en présence d'un liquide. Dans le sol, on placera l'électrode dans une région dont le pH est connu pour mesurer le potentiel libre ou potentiel de circuit ouvert. En reproduisant cette mesure pour différents pH, on obtient une calibration de l'électrode et du capteur, et le capteur peut être utilisé dans tous les milieux.

Le dispositif 100 comporte également un élément métallique 104, typiquement formé dans un matériau qui est préférentiellement le même que celui de la canalisation pour laquelle on souhaite réaliser une mesure.

L'élément métallique pourra également être désigné comme un coupon métallique ou un coupon gravimétrique par l'homme du métier.

Cet élément comporte une surface annulaire 105 qui entoure la surface 102 décrite ci-avant.

En fait, les surfaces 105 et 102 sont agencées toutes les deux vers le bas sur la figure, au milieu d'une première ouverture 106 d'un corps 107 dans lequel sont placés le capteur de pH et l'élément métallique. Le corps peut être formé dans un matériau polymérique, et être par exemple en polypropylène. Le corps 107 a ici une forme substantiellement cylindrique à base circulaire, mais d'autres formes sont possibles.

Les deux surfaces 105 et 102 sont donc laissées libres de sorte que si le dispositif est inséré dans le sol, ces surfaces seront en contact avec le fluide contenu dans le sol, ainsi, la surface 102 sera utilisée à des fins de mesure de pH.

Pour que cette mesure puisse bien illustrer la valeur du pH que l'on pourrait mesurer directement à proximité d'une canalisation enterrée à laquelle un potentiel électrique a été appliqué, on applique également un potentiel électrique à l'élément métallique 104. De ce fait, la surface 105 pourra affecter le pH de la même manière qu'au voisinage de la canalisation, ce qui rend la mesure par le capteur 101 très proche de celle que l'on pourrait faire à proximité de la canalisation enterrée.

On notera que les surfaces 102 et 105 sont en retrait par rapport à la surface S du corps, ce retrait ayant une profondeur notée r de l'ordre d'un millimètre, pour protéger les deux surfaces.

Pour appliquer ce potentiel électrique, on utilise des moyens de connexion électrique comprenant :
- un câble 108 pour amener un potentiel électrique à l'élément métallique 104,
- une fiche 109 montée à l'extrémité du câble qui est dans le dispositif, et
- un connecteur 110, par exemple en laiton recouvert de nickel, pressé par vissage contre l'élément métallique 104.

On notera que pour éviter que du fluide ne pénètre à l'intérieur du corps, un joint 111 est agencé autour de la surface 105, entre l'élément métallique 104 et une portion du corps 107contre laquelle le joint est maintenu. Le joint 111 forme des premiers moyens de maintien de l'étanchéité.

Pour pouvoir lire la valeur du pH fournie par le capteur 101, on utilise également des moyens de connexion électrique comprenant :
- un câble 112, et
- une fiche 113 insérée dans le capteur 101.

Les deux câbles 108 et 112 sont réunis dans une gaine 114 pour former un faisceau de câbles, et les deux extrémités de ces câbles 108 et 112 qui ne sont pas dans le corps 107 sont connectées respectivement à des fiches 114 et 115, par exemple des fiches selon la norme CEI 61010.

Les deux câbles 108 et 112 réunis dans la gaine 114 sortent du corps 107 par une deuxième ouverture 116 du corps, et le corps est fermé par un bouchon 117, par exemple en polypropylène, et ouvert pour laisser passer les deux câbles 108 et 112 réunis dans la gaine 114 à travers un presse-étoupe 118 qui formera des deuxièmes moyens de maintien de l'étanchéité.

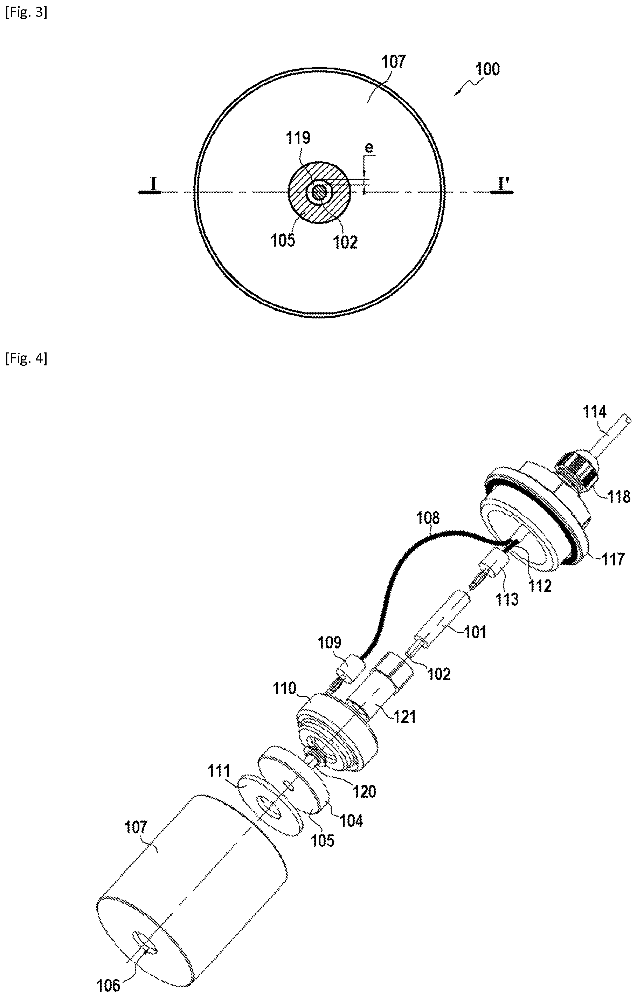

La figure 3 est une vue de dessous du dispositif 100 décrit en référence à la figure 2. Sur cette figure, on voit la surface 102 en polypyrrole qui a une forme circulaire et qui est placée au centre du dispositif. La surface 102 est entourée par la surface 105 en forme annulaire.

Sur cette figure, on voit également le plan de coupe I-I' correspondant à la figure 2.

À titre indicatif, la surface 102 a une aire égale à 0,07cm². La surface 105 a une aire égale à 1cm². Des aires plus grandes sont envisageables pour ces deux surfaces.

De manière avantageuse, une bande 119, de forme annulaire, sépare les surfaces 102 et 105. Cette bande a une épaisseur e.

La figure 4 est une vue en éclaté du dispositif 100 décrit en référence aux figures 2 et 3. Sur cette figure, on voit en outre que le capteur 101 est inséré dans un fourreau 120 à son extrémité comportant la surface 102, et dans une bride de fixation 121 isolante au niveau du connecteur 110.

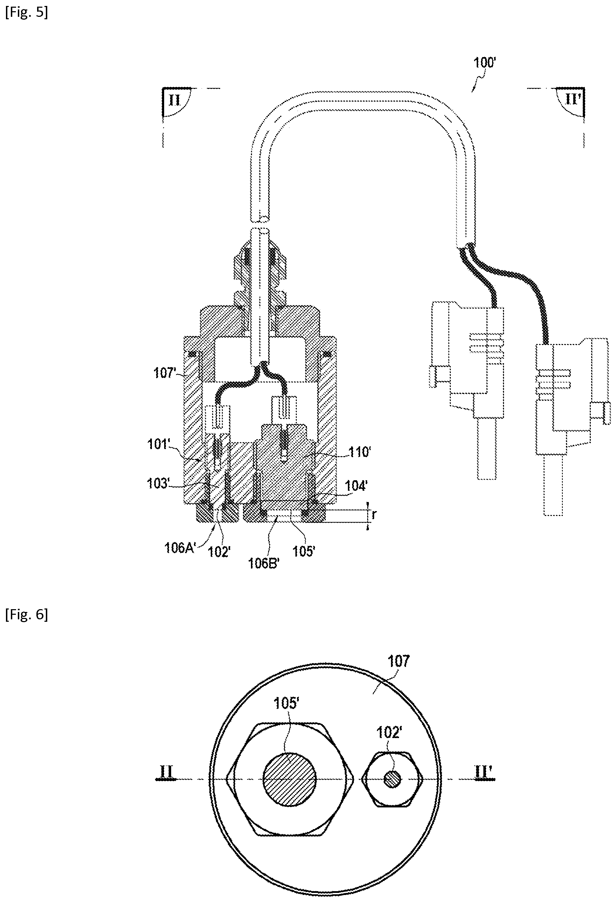

La figure 5 est une vue en coupe d'un dispositif 100', qui diffère de celui des figures 2 à 4 en ce que la surface de l'élément métallique et la surface comprenant du polypyrrole sont adjacentes sans que celle métallique n'entoure celle comprenant du polypyrrole. Les éléments identiques à ceux décrits en référence aux figures 2 à 4 ne seront pas décrits en relation avec le dispositif 100' par soucis de concision.

Plus précisément, le dispositif 100' comporte un capteur de pH 101' avec une surface 102' comprenant du polypyrrole. Le capteur 101' est identique au capteur 101 sauf en ce qu'il est placé contre une paroi du corps 107' du dispositif 100', et inséré directement dans le corps 107.

Le dispositif 100' comporte également un élément métallique 104' muni d'une surface 105. L'élément métallique 104' est agencé pour être diamétralement opposé au capteur de pH 101'. Un connecteur en laiton recouvert de nickel 110' est également utilisé pour recevoir le potentiel électrique de la même manière qu'en utilisant le connecteur 110 du dispositif 100. Le connecteur 110' est monté dans le corps 107'. Des connecteurs comprenant d'autres matériaux pourraient être utilisés, par exemple d'autre connecteurs conducteurs.

Ici, le corps 107 comporte deux premières ouvertures 106A' et 106B' qui reçoivent respectivement les surfaces 102' et 105'.

Les surfaces 102' et 105' sont en retrait par rapport à la surface S' du corps, avec un retrait ayant une profondeur r de l'ordre d'un millimètre.

La figure 6 est une vue de dessous du dispositif 100' décrit en référence à la figure 5. Sur cette figure, on voit la surface 102' en polypyrrole qui a une forme circulaire et qui est placée à droite sur la figure. La surface 102' est adjacente à la surface 105' en forme de disque et qui est placée à gauche sur la figure.

Sur cette figure, on voit également le plan de coupe II-II' correspondant à la figure 5.

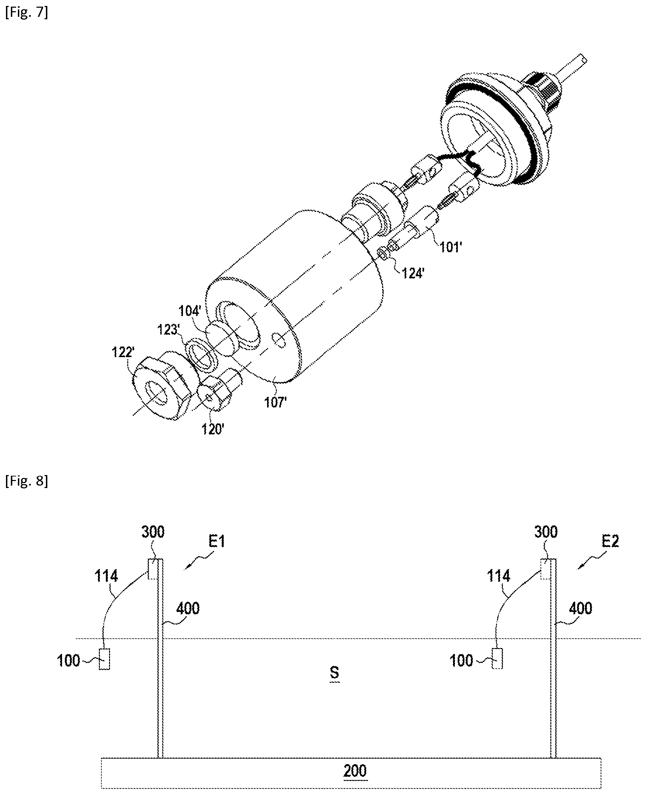

La figure 7 est une vue en éclaté du dispositif 100' décrit en référence aux figures 5 et 6. Sur cette figure, on voit en outre que le capteur 101' est inséré dans un fourreau 120 à son extrémité comportant la surface 102. Ici, le fourreau est en partie saillant par rapport au corps 107'.

Un fourreau 122' est également utilisé pour l'élément métallique 104'.

Par ailleurs, dans ce mode de réalisation, on utilise deux joints 123' et 124' respectivement associés au capteur de pH 101' et à l'élément métallique 104'.

Sur la figure 8, on a représenté une installation comprenant une canalisation 200 qui est enterrée dans le sol S. A un premier emplacement E1, une station de mesure, ou prise de potentiel 300 est installée pour être connectée à la canalisation 200 par un câble 400. A un deuxième emplacement E2, une station de mesure, ou prise de potentiel 300 est également installée pour être connectée à la canalisation 200 par un câble 400.

Généralement, ces prises de potentiel sont utilisées pour mesurer le potentiel électrique de la canalisation à laquelle on a appliqué un potentiel électrique à des fins de protection cathodique. Ces prises sont par exemple disposées tous les 4 kilomètres.

On peut utiliser des dispositifs 100 équipés de capteurs de pH tels que ceux décrits ci-avant en référence aux figures 2 à 4 pour mesurer le pH du sol aux emplacements E1 et E2. En fait, il est délicat d'atteindre la canalisation 200 pour effectuer une mesure. Par contre, on peut, à faible profondeur, insérer les dispositifs 100 dans le sol pour qu'ils soient en contact avec le même environnement que celui de la canalisation.

On utilise ici les prises de potentiel 300 pour appliquer des potentiels électriques directement appliqués sur la canalisation aux éléments métalliques des dispositifs 100, notamment en utilisant les câbles gainés 114.

Ces mesures permettent de suivre l'évolution du pH à différentes régions, ce qui permet ensuite de déterminer la valeur du potentiel électrique à appliquer à la canalisation 200 pour éviter la corrosion, par exemple en utilisant un diagramme de Pourbaix.

## Revendications

1. Procédé de mesure du pH d'un fluide contenu dans une région du sol contenant un fluide du sol dont on souhaite connaître le pH, comprenant :
- une insertion d'un dispositif à capteur de pH dans le sol pour être en contact avec la région contenant un fluide du sol dont on souhaite connaître le pH, le dispositif comprenant :
- un capteur de pH (101) comprenant une surface (102),
- un élément métallique (104) muni d'une surface (105) configurée pour être en contact avec ladite région contenant le fluide lorsque le dispositif est inséré dans le sol,
- des moyens de connexion électrique (108, 109, 110) connectés au moins à l'élément métallique et configurés pour recevoir un potentiel électrique de protection cathodique pour l'élément métallique,
l'insertion étant mise en œuvre de sorte que ladite surface (105) de l'élément métallique et ladite surface (102) se retrouvent en contact avec du fluide contenu dans une région du sol
- une application d'un potentiel électrique de protection cathodique à l'élément métallique par les moyens de connexion électrique (108, 109, 110), et,
- une mesure du pH au moyen du capteur de pH (101),
dans lequel le dispositif comprend un corps (107) muni d'au moins une première ouverture (106) configurée pour laisser, lorsque le dispositif est inséré dans le sol, le fluide contenu dans ladite région être en contact avec ladite surface (102) et avec ladite surface (105) de l'élément métallique,
et au moins une deuxième ouverture (116) configurée pour recevoir lesdits moyens de connexion électrique,
le procédé de mesure étant **caractérisé en ce que** la surface (102) comprend un matériau polymère capable d'attirer les protons et est agencée pour que le matériau polymère soit en contact avec ladite région contenant un fluide lorsque le dispositif est inséré dans le sol, et **en ce que** ladite surface (102) comprenant un matériau polymère et ladite surface (105) de l'élément métallique sont agencées en retrait par rapport à une surface externe du corps où sont formées les ouvertures.

2. Procédé selon la revendication 1, dans lequel le polymère contient un ou plusieurs groupes aminos.

3. Procédé selon la revendication 2, dans lequel le polymère est choisi dans le groupe comprenant le polypyrrolle et le poly(3,4-éthylènedioxythiophène).

4. Procédé selon la revendication 1, dans lequel le dispositif comprend des premiers moyens (111) de maintien de l'étanchéité agencés à l'interface entre d'une part ladite surface (102) comprenant un matériau polymère et ladite surface (105) de l'élément métallique et d'autre part le corps, pour empêcher le fluide contenu dans ladite région de pénétrer à l'intérieur du corps.

5. Procédé selon l'une quelconque des revendications 1 ou 4, dans lequel le dispositif comprend des deuxièmes moyens (118) de maintien de l'étanchéité agencés à l'interface entre d'une part les moyens de connexion électrique et d'autre part le corps, pour empêcher le fluide contenu dans ladite région de pénétrer à l'intérieur du corps.

6. Procédé selon l'une quelconque des revendications 1, 4 ou 5, dans lequel le capteur de pH et/ou l'élément métallique sont amovibles par rapport au corps.

7. Procédé selon l'une quelconque des revendications 1 à 6, dans lequel ladite surface (102) comprenant un matériau polymère et ladite surface (105) de l'élément métallique sont espacées par une bande ayant une épaisseur comprise entre 0,5 et 1,5 millimètre.

8. Procédé selon l'une quelconque des revendications 1 à 7, dans lequel ladite surface (105) de l'élément métallique a une forme annulaire et ladite surface (102) comprenant un matériau polymère est agencée de manière à être entourée par ladite surface (105) de l'élément métallique.

9. Procédé selon l'une quelconque des revendications 1 à 8, dans lequel ladite surface (105) de l'élément métallique a une aire au moins supérieure ou égale à 1 cm² et ladite surface (102) comprenant un matériau polymère a une aire au moins supérieure ou égale à 0,07 cm².

10. Procédé de protection cathodique d'une canalisation comprenant :
- une mise en œuvre du procédé de mesure du pH selon l'une quelconque des revendications 1 à 9 pour une région du sol située au-dessus d'une canalisation,
- une détermination d'une nouvelle valeur de potentiel électrique à appliquer à ladite canalisation à partir du résultat de ladite mesure du pH.

11. Dispositif à capteur de pH, destiné à être inséré dans le sol pour être en contact avec une région contenant un fluide du sol dont on souhaite connaître le pH, comprenant :
- un capteur de pH (101) comprenant une surface (102),
- un élément métallique (104) muni d'une surface (105) configurée pour être en contact avec ladite région contenant le fluide lorsque le dispositif est inséré dans le sol,
- des moyens de connexion électrique (108, 109, 110) connectés au moins à l'élément métallique et configurés pour recevoir un potentiel électrique de protection cathodique pour l'élément métallique,
dans lequel le dispositif comprend un corps (107) muni d'au moins une première ouverture (106) configurée pour laisser, lorsque le dispositif est inséré dans le sol, le fluide contenu dans ladite région être en contact avec ladite surface (102) et avec ladite surface (105) de l'élément métallique,
et au moins une deuxième ouverture (116) configurée pour recevoir lesdits moyens de connexion électrique,
le dispositif à capteur de pH étant **caractérisé en ce que** la surface (102) comprend un matériau polymère capable d'attirer les protons et est agencée pour que le matériau polymère soit en contact avec ladite région contenant un fluide lorsque le dispositif est inséré dans le sol, et **en ce que** ladite surface (102) comprenant un matériau polymère et ladite surface (105) de l'élément métallique sont agencées en retrait par rapport à une surface externe du corps où sont formées les ouvertures.

## Patentansprüche

1. Verfahren zum Messen des pHs eines Fluids, das in einem Bodenbereich enthalten ist, der ein Fluid des Bodens enthält, dessen pH gekannt werden soll, umfassend:
- ein Einführen einer pH-Sensorvorrichtung in den Boden, um mit dem Bereich in Kontakt zu sein, der ein Fluid des Bodens enthält, dessen pH gekannt werden soll, die Vorrichtung umfassend:
- einen pH-Sensor (101), umfassend eine Oberfläche (102),
- ein Metallelement (104), das mit einer Oberfläche (105), versehen ist, die konfiguriert ist, um mit dem Bereich in Kontakt zu sein, der das Fluid enthält, wenn die Vorrichtung in den Boden eingeführt ist,
- elektrische Verbindungseinrichtungen (108, 109, 110), die zumindest mit dem Metallelement verbunden und konfiguriert sind, um ein elektrisches Potential zum kathodischen Schutz für das Metallelement zu empfangen,
wobei das Einführen durchgeführt wird, sodass die Oberfläche (105) des Metallelements und die Oberfläche (102) in Kontakt mit einem Fluid kommen, das in einem Bereich des Bodens enthalten ist,
- ein Anlegen eines elektrischen Potenzials zum kathodischen Schutz an das Metallelement durch die elektrischen Verbindungseinrichtungen (108, 109, 110), und
- eine Messung des pHs mittels des pH-Sensors (101),
wobei die Vorrichtung einen Körper (107) umfasst, der mit mindestens einer ersten Öffnung (106) versehen ist, die konfiguriert ist, um, wenn die Vorrichtung in den Boden eingeführt ist, das in diesem Bereich enthaltene Fluid mit der Oberfläche (102) und der Oberfläche (105) des Metallelements in Kontakt zu lassen,
und mindestens eine zweite Öffnung (116), die konfiguriert ist, um die elektrischen Verbindungseinrichtungen aufzunehmen,
wobei das Messverfahren **dadurch gekennzeichnet ist, dass** die Oberfläche (102) ein polymeres Material umfasst, das in der Lage ist, die Protonen anzuziehen, und ausgebildet ist, damit das polymere Material mit dem Bereich in Kontakt ist, der ein Fluid enthält, wenn die Vorrichtung in den Boden eingeführt ist, und dass die Oberfläche (102), die ein polymeres Material umfasst, und die Oberfläche (105) des Metallelements in Bezug auf eine äußere Oberfläche des Körpers, an der die Öffnungen gebildet sind, vertieft ausgebildet sind.

2. Verfahren nach Anspruch 1, wobei das Polymer eine oder mehrere Aminogruppen enthält.

3. Verfahren nach Anspruch 2, wobei das Polymer ausgewählt ist aus der Gruppe, bestehend aus Polypyrrol und Poly(3,4-ethylendioxythiophen).

4. Verfahren nach Anspruch 1, wobei die Vorrichtung erste Dichthalteeinrichtungen (111) umfasst, die an der Grenzfläche zwischen einerseits der Oberfläche (102), die ein polymeres Material umfasst, und der Oberfläche (105) des Metallelements und andererseits dem Körper ausgebildet sind, um zu verhindern, dass das in dem Bereich enthaltene Fluid in das Innere des Körpers eindringt.

5. Verfahren nach einem der Ansprüche 1 oder 4, wobei die Vorrichtung zweite Dichthalteeinrichtungen (118) umfasst, die an der Schnittstelle zwischen einerseits den elektrischen Verbindungseinrichtungen und andererseits dem Körper ausgebildet sind, um zu verhindern, dass das in diesem Bereich enthaltene Fluid in das Innere des Körpers eindringt.

6. Verfahren nach einem der Ansprüche 1, 4 oder 5, wobei der pH-Sensor und/oder das Metallelement in Bezug auf den Körper abnehmbar sind.

7. Verfahren nach einem der Ansprüche 1 bis 6, wobei die Oberfläche (102), die ein polymeres Material umfasst, und die Oberfläche (105) des Metallelements durch einen Streifen mit einer Stärke zwischen 0,5 und 1,5 Millimetern voneinander beabstandet sind.

8. Verfahren nach einem der Ansprüche 1 bis 7, wobei die Oberfläche (105) des Metallelements eine ringförmige Gestalt aufweist und die Oberfläche (102), die ein polymeres Material umfasst, ausgebildet ist, um von der Oberfläche (105) des Metallelements umgeben zu sein.

9. Verfahren nach einem der Ansprüche 1 bis 8, wobei die Oberfläche (105) des Metallelements eine Fläche von mindestens größer als oder gleich wie 1 cm² aufweist, und die Oberfläche (102), die ein polymeres Material umfasst, eine Fläche von mindestens größer als oder gleich wie 0,07 cm² aufweist.

10. Verfahren zum kathodischen Schutz einer Rohrleitung, umfassend:
- ein Durchführen des Verfahrens zum Messen des pHs nach einem der Ansprüche 1 bis 9 für einen Bereich des Bodens, der sich oberhalb einer Rohrleitung befindet,
- ein Bestimmen eines neuen Werts des elektrischen Potenzials, das an die Rohrleitung angelegt werden soll, anhand des Resultats der pH-Messung.

11. pH-Sensorvorrichtung, die dazu bestimmt ist, in den Boden eingeführt zu werden, um mit einem Bereich in Kontakt zu sein, der ein Fluid des Bodens enthält, dessen pH gekannt werden soll, umfassend:
- einen pH-Sensor (101), umfassend eine Oberfläche (102),
- ein Metallelement (104), das mit einer Oberfläche (105), versehen ist, die konfiguriert ist, um mit dem Bereich in Kontakt zu sein, der das Fluid enthält, wenn die Vorrichtung in den Boden eingeführt ist,
- elektrische Verbindungseinrichtungen (108, 109, 110), die zumindest mit dem Metallelement verbunden und konfiguriert sind, um ein elektrisches Potential zum kathodischen Schutz für das Metallelement zu empfangen,
wobei die Vorrichtung einen Körper (107) umfasst, der mit mindestens einer ersten Öffnung (106) versehen ist, die konfiguriert ist, um, wenn die Vorrichtung in den Boden eingeführt ist, das in diesem Bereich enthaltene Fluid mit der Oberfläche (102) und der Oberfläche (105) des Metallelements in Kontakt zu lassen,
und mindestens eine zweite Öffnung (116), die konfiguriert ist, um die elektrischen Verbindungseinrichtungen aufzunehmen,
wobei die pH-Sensorvorrichtung **dadurch gekennzeichnet ist, dass** die Oberfläche (102) ein polymeres Material umfasst, das in der Lage ist, die Protonen anzuziehen, und ausgebildet ist, damit das polymere Material mit dem Bereich in Kontakt ist, der ein Fluid enthält, wenn die Vorrichtung in den Boden eingeführt ist, und dass die Oberfläche (102), die ein polymeres Material umfasst, und die Oberfläche (105) des Metallelements in Bezug auf eine äußere Oberfläche des Körpers, an der die Öffnungen gebildet sind, vertieft ausgebildet sind.

## Claims

1. A method for measuring the pH of a fluid contained in a region of soil containing a soil fluid the pH of which is to be known, comprising:
- inserting a pH sensor device into the soil so as to be in contact with the region containing a soil fluid the pH of which needs to be known, the device comprising:
- a pH sensor (101) comprising a surface (102),
- a metallic element (104) equipped with a surface (105) configured to be in contact with said region containing the fluid when the device is inserted into the soil,
- means of electrical connection (108, 109, 110) connected at least to the metallic element and configured to receive a cathodic protection electrical potential for the metallic element, the insertion being implemented so that said surface (105) of the metallic element and said surface (102) are in contact with fluid contained in a region of soil
- applying a cathodic protection electrical potential to the metallic element by means of the electrical connection (108, 109, 110), and,
- measuring the pH by means of the pH sensor (101),
wherein the device comprises a body (107) equipped with at least one first opening (106) configured to allow, when the device is inserted into the soil, the fluid contained in said region to be in contact with said surface (102) and with said surface (105) of the metallic element,
and at least one second opening (116) configured to receive said means of electrical connection,
the measurement method being **characterized in that** the surface (102) comprises a polymer material able to attract protons and is arranged so that the polymer material is in contact with said region containing a fluid when the device is inserted into the soil, and **in that** said surface (102) comprising a polymer material and said surface (105) of the metallic element are arranged, recessed, relative to an external surface of the body wherein the openings are formed.

2. The method according to claim 1, wherein the polymer contains one or more amino groups.

3. The method according to claim 2, wherein the polymer is selected from the group comprising polypyrrole and poly(3,4-ethylenedioxythiophene).

4. The method according to claim 1, wherein the device comprises first means (111) for maintaining the seal, arranged at the interface between, on the one hand, said surface (102) comprising a polymer material and said surface (105) of the metallic element and, on the other hand, the body, to prevent the fluid contained in said region from penetrating into the interior of the body.

5. The method according to any one of claims 1 or 4, wherein the device comprises second means (118) for maintaining the seal arranged at the interface between, on the one hand, the means of electrical connection and, on the other hand, the body, to prevent the fluid contained in said region from penetrating into the interior of the body.

6. The method according to any one of claims 1, 4 or 5, wherein the pH sensor and/or the metallic element are removable relative to the body.

7. The method according to any one of claims 1 to 6, wherein said surface (102) comprising a polymer material and said surface (105) of the metallic element are spaced apart by a strip having a thickness between 0.5 and 1.5 millimeters.

8. The method according to any one of claims 1 to 7, wherein said surface (105) of the metallic element has an annular shape and said surface (102) comprising a polymer material is arranged so as to be surrounded by said surface (105) of the metallic element.

9. The method according to any one of claims 1 to 8, wherein said surface (105) of the metallic element has an area at least greater than or equal to 1 cm² and said surface (102) comprising a polymer material has an area at least greater than or equal to 0.07 cm².

10. A cathodic protection method for a pipeline comprising:
- implementing the pH measurement method according to any one of claims 1 to 9 for a region of soil located above a pipeline,
- determining a new value of electrical potential to be applied to said pipeline based on the result of said pH measurement.

11. A pH sensor device, intended to be inserted into the soil to be in contact with a region containing a soil fluid the pH of which needs to be known, comprising:
- a pH sensor (101) comprising a surface (102),
- a metallic element (104) equipped with a surface (105) configured to be in contact with said region containing the fluid when the device is inserted into the soil,
- means of electrical connection (108, 109, 110) connected at least to the metallic element and configured to receive a cathodic protection electrical potential for the metallic element,
wherein the device comprises a body (107) equipped with at least one first opening (106) configured to allow, when the device is inserted into the soil, the fluid contained in said region to be in contact with said surface (102) and with said surface (105) of the metallic element, and at least one second opening (116) configured to receive said means of electrical connection, the pH sensor device being **characterized in that** the surface (102) comprises a polymer material able to attract protons and is arranged so that the polymer material is in contact with said region containing a fluid when the device is inserted into the soil, and **in that** said surface (102) comprising a polymer material and said surface (105) of the metallic element are arranged, recessed, relative to an external surface of the body wherein the openings are formed.
